(19) Europäisches Patentamt / European Patent Office / Office européen des brevets

(11) **EP 3 012 242 B1**

(12) **EUROPEAN PATENT SPECIFICATION**

(45) Date of publication and mention
of the grant of the patent:
**13.02.2019 Bulletin 2019/07**

(21) Application number: **14812985.1**

(22) Date of filing: **26.03.2014**

(51) Int Cl.:
*C07C 29/76* (2006.01)  *C07C 29/78* (2006.01)
*C07C 29/80* (2006.01)  *C07C 35/12* (2006.01)
*C09B 11/00* (2006.01)  *C09B 11/02* (2006.01)

(86) International application number:
**PCT/JP2014/058641**

(87) International publication number:
**WO 2014/203580 (24.12.2014 Gazette 2014/52)**

(54) **PURIFIED NATURAL (L)-MENTHOL, MANUFACTURE METHOD AND EVALUATION METHOD THEREFOR**

NATÜRLICHES (L)-MENTHOLGEREINIGTES PRODUKT SOWIE HERSTELLUNGSVERFAHREN UND BEURTEILUNGSVERFAHREN DAFÜR

PRODUIT PURIFIFIÉ DE (L)-MENTHOL NATUREL ET PROCÉDÉ DE PRODUCTION ET PROCÉDÉ D'ÉVALUATION S'Y RAPPORTANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2013 JP 2013127966**

(43) Date of publication of application:
**27.04.2016 Bulletin 2016/17**

(73) Proprietors:
• **Japan Tobacco Inc.**
  **Tokyo 105-8422 (JP)**
• **Inabata Koryo Co., Ltd.**
  **Osaka-shi, Osaka 532-0027 (JP)**

(72) Inventors:
• **AKIYAMA, Shintaro**
  **Tokyo 130-8603 (JP)**
• **YOSHIOKA, Yuri**
  **Osaka-shi**
  **Osaka 532-0027 (JP)**
• **YASUDA, Koji**
  **Osaka-shi**
  **Osaka 532-0027 (JP)**
• **MATSUMURA, Shinichi**
  **Osaka-shi**
  **Osaka 532-0027 (JP)**

(74) Representative: **Isarpatent**
  **Patent- und Rechtsanwälte Behnisch Barth Charles**
  **Hassa Peckmann & Partner mbB**
  **Friedrichstrasse 31**
  **80801 München (DE)**

(56) References cited:
  **JP-A- 2012 517 993    US-A1- 2008 228 013**

• **'Heisei 18 Nendo Hyojun Gijutsushu Koryo' JAPANESE PATENT OFFICE 2007, pages 238 - 240, XP008179671**
• **'Koryo Kagaku Soran [II' KABUSHIKI KAISHA 15 January 1968, pages 556 - 566, XP008179673**
• **HEISEI 18 NENDO HYOJUN GIJUTSUSHU KORYO 2007, pages 565 - 570, XP008179672**

**Description**

[TECHNICAL FIELD]

The present invention relates to a method for manufacturing a purified natural (L)-menthol, and more particularly, to a method for manufacturing a purified natural (L)-menthol for smoking, with unpleasant taste eliminated and a refreshing feeling added by addition of activated carbon to crude menthol.

[BACKGROUND ART]

Menthol is an organic compound, a kind of a cyclic monoterpene alcohol, and is a mint-smelling volatile colorless crystal. Some menthol exist in diastereomer and enantiomer, and natural (L)-menthol is widely used not only for toothpastes, confectionery, mouth refrigerants, etc. but also for pharmaceuticals, due to its partial vasodilator action and skin irritant action etc.

Menthol has been conventionally used as flavor of tobacco. When raw leaf tobacco is burned, it has a peculiar stimulus, off smell such as green smelling or fishy smelling, unpleasant taste such as astringency and bitterness, addition to its peculiar smoking flavor and taste. Thus, licorice, sugar, honey, rum, fruit juice, vanilla, etc. have been empirically used to soften off smell and unpleasant taste.

People's preference in tobacco taste has recently become diverse and products with refreshing and rich smoking flavor and taste have been increasingly popular. Among ingredients contained in a so called modifier for tobacco flavor and taste, particularly natural (L)-menthol plays a large role.

As a menthol, natural (L)-menthol and synthetic (L)-menthol are used. Synthetic (L)-menthol is obtained in higher purity than natural (L)-menthol, but is inferior to natural (L)-menthol in terms of flavor, since it does not contain flavor ingredient impurities derived from raw mint oil. Natural (L)-menthol is extracted from a plant, such as a mint leaf, and thus contains impurities. The content of this impurities affects quality and flavor of the natural (L)-menthol. For that reason, methods of purifying natural (L)-menthol have been considered from various aspects. One of them includes a method for contacting crude menthol with supercritical carbon dioxide, and for fractionating, extracting, and removing impurities (for example, see patent document 1). This method can remove ingredients such as unpleasant smell (taste), bitter components, weed-like smell, substances with strongly unfavorable taste and resinoid substances. However, this method utilizes supercritical carbon dioxide and thus requires expensive devices. Accordingly, the method is hard to industrialize.

One of other methods includes a method for dissolving crude menthol in acetonitrile and then cooling the solution to crystallize the menthol (for example, see patent document 2). This method yields natural (L)-menthol with more than 99% of chemical purity and more than 99%ee of optical purity, but working environment to implement the method is limited since it requires that menthol should be dissolved in an organic solvent (acetonitrile).

As described above, even if using the art indicated by the patent document 1 or 2, it would be difficult to easily and inexpensively provide a purified natural (L)-menthol with a refreshing feeling and mint-like flavor.

Gas chromatography has been traditionally used as an evaluation method for measuring degree of the purification of purified natural (L)-menthol. However, the method had a problem of associating a difference in components and amounts of impurities in the natural (L)-menthol with people's preference, since a very little amount of the impurities is contained in the natural (L)-menthol.

[PRIOR ART DOCUMENTS]

[PATENT LITERATURE]

[Patent document 1] Japanese Patent No. 3935987
[Patent document 2] Japanese Patent No. 5014846
US-A-2008228013 describes a method for manufacturing a purified (L)-menthol comprising the step of precipitating the desired compound from a solution of crude menthol in acetonitrile. A purified (L)-menthol having a purity higher than 99% and an optical purity of 99,6% e.e. is obtained.

[SUMMARY OF THE INVENTION]

[PROBLEMS TO BE SOLVED BY THE INVENTION]

The present invention aims to solve the above-described problems of the prior art and to provide a method for manufacturing a purified natural (L)-menthol with unpleasant taste eliminated and having a refreshing feeling and mint-like flavor derived from natural products.

[MEANS TO SOLVE THE PROBLEMS]

The invention according to claim 1 relates to a method for manufacturing a purified natural (L)-menthol, comprising; a step of heating and melting crude menthol containing 90% or more of the purified natural (L)-menthol to obtain a liquid crude menthol, a step of adding activated carbon to the liquid crude menthol, and a step of stirring the obtained liquid menthol to make the activated carbon absorb impurities in the crude menthol.

The invention according to claim 2 relates to the method for manufacturing a purified natural (L)-menthol of claim 1, further comprising; a step of filtering the obtained liquid menthol with the activated carbon absorbing the impurities to remove the activated carbon and a step of cooling down and recrystallizing the menthol.

Described but not claimed *per se* is a purified natural (L)-menthol with a refreshing feeling and mint-like flavor which chroma measured by following steps is equal to or less than 1.

(1) a step of subjecting the purified natural (L)-menthol in heated and melted state to distillation under reduced pressure to obtain a residue distilled under reduced pressure.
(2) a step of adding ethanol to the residue obtained at the step (1) to prepare an ethanol solution.
(3) a step of calculating chroma with a color difference meter pursuant to ISO 7724 (JIS Z8722) using the ethanol solution obtained at the step (2) as a color difference measurement sample.

The purified natural (L)-menthol as described above may be with the above-mentioned chroma within a range of 0.4 to 0.6.

The purified natural (L)-menthol as described above may contain the residue distilled under reduced pressure of the above-mentioned purified natural (L)-menthol in an amount of less than 0.1 % by weight.

Additionally described but not claimed *per se* is a method for evaluating a purified natural (L)-menthol, characterizing by measuring chroma by the following steps;

(1) a step of subjecting the purified natural (L)-menthol in heated and melted state to distillation under reduced pressure to obtain a residue distilled under reduced pressure.
(2) a step of adding ethanol to the residue obtained at the step (1) to prepare an ethanol solution.
(3) a step of calculating chroma with a color difference meter pursuant to ISO 7724 (JIS Z8722) using the ethanol solution obtained at the step (2) as a color difference measurement sample.

[EFFECT OF THE INVENTION]

According to the invention of claim 1, the method can effectively remove impurities by comprising a step of contacting the heated and melted crude menthol containing 90% or more of the purified natural (L)-menthol with activated carbon. The method enables the purified natural (L)-menthol to be more easily, inexpensively, and stably obtained with a refreshing feeling and mint-like flavor, compared to the conventional methods. Removal of appropriate amount of the impurities with the activated carbon enables the purified natural (L)-menthol to be easily purified with a refreshing feeling and mint-like flavor.

According to the invention of claim 2, the liquid menthol obtained based on the invention of claim 4 is filtered to remove the activated carbon and then recrystallized to obtain a purified natural (L)-menthol.

Regarding the purified natural (L)-menthol which is described above but not claimed *per se,* measuring an ethanol solution prepared by adding ethanol to the residue after the distillation under reduced pressure of purified natural (L)-menthol using a color difference meter pursuant to ISO 7724 enables confirmation of the existence of impurities which has big influence on people's preference. In such a case, it is possible to prepare the purified natural (L)-menthol having a refreshing feeling and mint-like flavor from the purified natural (L)-menthol which chroma is equal to or less than 1.

Moreover, when the chroma of the purified natural (L)-menthol evaluated by a color difference meter in a similar method as described above is within a range of 0.4 to 0.6, the purified natural (L)-menthol having a refreshing feeling and mint-like flavor stronger than those of the purified natural (L)-menthol of claim 1 could be prepared (see Table 1).

Further, since the residue distilled under reduced pressure of the above-described purified natural (L)-menthol can be less than 0.1 % by weight, it is possible to obtain a purified natural (L)-menthol having a refreshing feeling and mint-like flavor with little impurities by a relatively easy step of contacting the purified natural (L)-menthol with activated carbon.

According to the method for evaluating a purified natural (L)-menthol, which is additionally described above but not claimed *per se,* it is possible to confirm the existence of impurities by measuring the ethanol solution obtained by adding ethanol to the residue distilled under reduced pressure of the purified natural (L)-menthol with a color difference meter pursuant to ISO 7724. This evaluation method enables confirmation of very little amount of impurities, which was impossible with the conventional methods such as gas chromatography used for quality measurement, and numerical evaluation of a refreshing feeling and mint-like flavor using chroma (see table 1).

[DETAILED DESCRIPTION OF THE INVENTION]

The method for manufacturing a purified natural (L)-menthol according to the present invention and the method for evaluating the purified natural (L)-menthol will be set forth in detail below.

1. Manufacturing Method of Purified natural (L)-menthol

Crude menthol according to the present invention is obtained by extraction, such as steam distillation, from Mentha aquatica, Mentha arvensis, Mentha longifolia, Mentha pulegium, Mentha spicata, Mentha piperita, etc. which belong to the genus of Mentha of the Lamiaceae family. A place of production and a kind of these materials are not specifically limited. The crude menthol obtained from the essential oil extracted through steam distillation, etc., from dried leaves and other shoots of these Mentha genus is used for the present invention.

For the purpose of effective purification, the crude menthol may better have as much (L)-menthol as possible, and preferably 90% or more of (L)-menthol. The extraction method is not specifically limited. For example, the crude menthol may be a natural (L)-menthol obtained by crystallization and centrifugation of essential oil, and be further refined by distillation, column chromatography, etc.

[Heating and Melting Process]

The extracted crude menthol is heated and melted into liquid crude menthol before addition of the activated carbon. Since the melting point of menthol is around 42 °C, the heating temperature may be, but not specifically limited to, 45-100 °C, preferably 55-65 °C.

[Adding Process of Activated Carbon]

The activated carbon is added to this liquid crude menthol, where the amount of activated carbon added may be 0.1 to 10 wt%, preferably 1 to 4 wt%, of the crude menthol. It is not preferable that the amount is less than 0.1 wt%, since the impurities in the crude menthol cannot be sufficiently removed and consequently the unfavorable taste cannot be removed. Also, it is not preferable that the amount exceeds 10 wt%, since the excessive impurities of the crude menthol are removed and the mint-like flavor is lost. The mint-like flavor refers to a flavor of natural mint.

[Stirring Process]

Then, the crude menthol is stirred with being heated in order to prevent from solidifying, so that the impurities are absorbed to the activated carbon. At this time, the temperature for heating the crude menthol may be in the range of 50 °C to 80 °C, preferably 55 °C to 65 °C, and the stirring time may be in the range of 30 minutes to 24 hours, preferably 2 to 5 hours. A heat retention means and a stirring means are not specifically limited to this.

[Removing Process of Activated Carbon]

Next, the stirred liquid menthol is filtered to remove the activated carbon. A filtering method and an apparatus to be used are not specifically limited in this process, and pressure filtration or filtration under reduced pressure can be used. In the Examples below, the press filter was used.

[Recrystallization Process]

The obtained filtrate is cooled down to be recrystallized. A cooling rate is, not specifically limited, but preferably slow, such that the temperature of the filtrate may be decreased, for example, at about 1-5 °C/min. For instance, a melting container is left under normal temperature to decrease the liquid temperature of the crude menthol melting solution, etc.

### 2. Evaluating Method

Next, an evaluating method of the purified natural (L)-menthol will be explained as follows.

In order to evaluate the smoking taste of the recrystallized purified natural (L)-menthol, it is added to a tobacco. For a comparison between samples, a certain amount of the purified menthol was added to each sample. The evaluation of the smoking taste consists of two evaluation criteria, refreshing feeling and mint-like flavor. Refreshing feeling refers to refreshing feeling which remains in the mouth after smoking, and the mint-like flavor refers to a flavor of natural mint. This purified natural (L)-menthol having refreshing feeling and mint-like flavor is blended into the tobacco to obtain a tasty menthol tobacco.

In order to measure an amount of the impurities contained in the above-described purified natural (L)-menthol, the purified menthol is heated and melted, and distilled under reduced pressure. The purified menthol is distilled at 2 kPa or less with the heating temperature of 80 °C . The heating temperature may be in the range of 75-95 °C. Ethanol is added to the obtained residue. This ethanol solution is used as a color difference measurement sample.

L*a*b* value of the color difference measurement sample is measured using a color difference meter according to ISO 7724 (JIS Z8722) to calculate chroma, thereby evaluating a degree of purification. The smoking taste correlates with the chroma, i.e., the higher the evaluation of the smoking taste is, the lower the chroma is. Thus, chroma of the purified natural (L)-menthol is preferably 1 or less, more preferably within the range of 0.4 to 0.6 (see Table 1).

When the purified menthol was used as a color difference measurement sample, there was little difference in chroma, and thus it was difficult to find out any correlation between the smoking taste and chroma. After the keen examination by the inventors with respect to the above-mentioned matter, the purified menthol was distilled under reduced pressure in a heating and melting state to obtain a residue. Then, the inventors added ethanol to the residue and used the ethanol solution as a color difference measurement sample to find out a correlation between the smoking taste and chroma is found out.

### 3. Purified Natural (L)-Menthol

The purified natural (L)-menthol is characterized in that chroma measured by the above-mentioned evaluating method is 1 or less. The purified natural (L) -menthol with the chroma being 1 or less can have refreshing feeling and mint-like flavor, wherein the chroma is preferably within the range of 0.4 to 0.6 to produce a purified natural (L)-menthol having stronger refreshing feeling and mint-like flavor. Also this purified natural (L)-menthol is blended with tobacco to obtain a tasty menthol tobacco (see Table 1).

The purified natural (L)-menthol does not depend on the (L)-menthol content, but it can be efficiently obtained by contacting the activated carbon with preferably 90% or more of crude menthol and filtering the menthol. Removing an appropriate amount of impurities by the activated carbon allows for easy purification of the purified natural (L)-menthol having refreshing feeling and mint-like flavor. After the removal of the activated carbon by filtration, the filtrate is recrystallized to finally obtain a purified natural (L) -menthol. The (L)-menthol content is preferably 97% or more, and more preferably 99% or more.

Also, the residue obtained by distillation under reduced pressure of the above-described purified natural (L)-menthol is preferably less than 0.1 wt%. This allows for a production of a purified natural (L) -menthol having refreshing feeling and mint-like flavor with little impurities by the relatively easy process of contacting the purified natural (L) -menthol with the activated carbon.

### [EXAMPLES]

Hereinafter, the effects of the present invention will be made clearer by illustrating Examples relating to the purified natural (L)-menthol, manufacturing method, and evaluating method. However, the present invention is not limited to the following Examples.

A naturally-derived crude menthol was heated, melted, and stirred after addition of the activated carbon. The activated carbon was then removed and the menthol was recrystallized from the filtrate to give a purified natural (L)-menthol. At this time, the obtained purified menthol went through sensory evaluation. The purified menthol was also distilled and ethanol was added to the residue. L*a*b* value was measured by setting this purified menthol as a color difference measurement sample to calculate chroma.

[Preparation Example 1]

500 g of crude menthol was heated and melted and activated carbon (0 wt%, 1 wt%, 2 wt%, 4 wt%) was added to the menthol. It was then stirred for 0, 2.5, 5, or 15 hours under hot bath at 60 °C to make the activated carbon absorb the impurities. It was then vacuum filtered by a 0.5 micrometer membrane filter to remove the activated carbon. The resulting filtrate was cooled down by being left under normal temperature and recrystallized. The purified Shirasagi (for brewing) made by Japan EnviroChemicals, Limited was used as activated carbon in this Example.

Next, production procedures of a filter cigarette using a purified natural (L)-menthol will be explained as follows. First, the obtained purified natural (L)-menthol was dissolved into a solvent solution to prepare a loading solution. Ethanol was used as a solvent solution. The loading solution may contain other perfumes as well as the natural (L)-menthol and the solvent solution is not limited to ethanol. Also, a blending ratio of a natural (L)-menthol and solvent solution may be, but not specifically limited to, 9:1-5:5. Then, the loading solution was added to shredded tobacco (American Blend, no perfume added after shredding) by a method such as spraying and use of a nozzle so that the (L)-menthol to shredded tobacco ratio is 10,000 ppm, and the shredded tobacco was processed to a filter cigarette. The menthol may be added not only to the shredded tobacco but also to a filter in the cigarette, but not specifically limited to them. With respect to a procedure for producing a filter cigarette using a natural (L)-menthol, the similar procedures were used in Preparation Examples 2 and 3.

Next, sensory evaluation of smoking taste of a cigarette will be explained as follows. The evaluation was performed by 14 trained expert assessors . The evaluation of the smoking taste consists of two evaluation criteria, intensity of refreshing feeling and quality and intensity of mint-like flavor. When the natural (L)-menthol having refreshing feeling and mint-like flavor is blended into a tobacco, a tasty tobacco product can be obtained.

250g of the purified natural (L)-menthol was distilled with 2 kPa or less at 80 °C. 20 ml of 95% ethanol was added to the resulting residue. L*a*b* value was measured by using this menthol as a color difference measurement sample using a colorimetric color difference meter ZE-2000 (made by Nippon Denshoku Industries Co., Ltd) pursuant to ISO 7724 (JIS Z8722) to calculate chroma. A transmission method was used as a measuring method. Results are shown in Table 1. The purified natural (L)-menthol with the chroma being more than 1 was described as Comparative Examples 1 and 2 and that with the chroma being 1 or less was described as Examples 1, 2, 3, and 4, respectively.

The Chroma can be obtained by calculating L*a*b*, which was measured by a colorimetric color difference meter, using the following formula. L*, a*, and b* (a combination of these three values refer to L*a*b* value) correspond to lightness of a color (L= 0 is black diffuse color and L= 100 is a white diffuse color), a position between red and green (the negative value of "a" is close to green and the positive value is close to red), and a position between yellow and blue (the negative value of "b" is close to blue and the positive value is close to yellow), respectively.

$$\text{Chroma } (C*) = \sqrt{(a*)^2 + (b*)^2}$$

[Preparation Example 2]

6,000 g of crude menthol was distilled with 2 kPa or less at 80 °C. The menthol concentrated to the weight of 3,000 g was used as a concentrated product having twice amounts of the impurities. Some distillates are used in Preparation Example 3.

500 g of this concentrated product having twice amounts of the impurities was heated and melted and activated carbon (0 wt%, 1 wt%, 2 wt%, 4 wt%) was added to the product. It was then stirred for 0, 2.5, 5, or 15 hours under hot bath at 60 °C to make the activated carbon absorb the impurities. It was then vacuum filtered by a 0.5 micrometer membrane filter to remove the activated carbon. The resulting filtrate was cooled down by being left under normal temperature and recrystallized.

The loading solution containing the obtained purified natural (L)-menthol was added to shredded tobacco so that the (L)-menthol to shredded tobacco ratio is 10,000 ppm, as shown in Preparation Example 1, and the shredded tobacco was processed to a filter cigarette (American Blend, no perfume added after shredding) . Then, it went through sensory evaluation of smoking taste. The evaluation of the smoking taste consists of two evaluation criteria, intensity of refreshing feeling and quality and intensity of mint-like flavor. When the natural (L)-menthol having refreshing feeling and mint-like flavor is blended into a tobacco product, a tasty tobacco product can be obtained.

250 g of crude menthol was also distilled with 2 kPa or less at 80 °C. 20 ml of 95% ethanol was added to the resulting residue. L*a*b* value was measured by using this menthol as a color difference measurement sample using a colorimetric color difference meter ZE-2000 (made by Nippon Denshoku Industries Co., Ltd) pursuant to ISO 7724 (JIS Z8722) to calculate chroma. Results are shown in Table 1. The purified natural (L)-menthol with the chroma being more than 1 was described as Comparative Examples 3, 4, 5, 6, and 7 and that with the chroma being 1 or less was described as Example 5, respectively.

[Preparation Example 3]

1,500 g of some distillates obtained in Preparation Example 2 and 1, 500 g of crude menthol were blended to give a concentrated product having 1/2 times amounts of the impurities.

500 g of this concentrated product having 1/2 times amounts of the impurities was heated and melted and activated carbon (0 wt%, 1 wt%, 2 wt%, 4 wt%) was added to the product. It was then stirred for 0, 2.5, 5, or 15 hours under hot bath at 60 °C to make the activated carbon absorb the impurities. It was then vacuum filtered by a 0.5 micrometer membrane filter to remove the activated carbon. The resulting filtrate was cooled down by being left under normal temperature and recrystallized.

The loading solution containing the obtained purified natural (L)-menthol was added to shredded tobacco so that the (L)-menthol to shredded tobacco ratio is 10,000 ppm, as shown in Preparation Examples 1 and 2, and the shredded tobacco was processed to a filter cigarette (American Blend, no perfume added after shredding). Then, it went through Sensory evaluation of smoking taste . The evaluation of the smoking taste consists of two evaluation criteria, intensity of refreshing feeling, and quality and intensity of mint-like flavor. When the natural (L) -menthol having refreshing feeling and mint-like flavor is blended into a tobacco product, a tasty tobacco product can be obtained.

250 g of crude menthol was also distilled with 2 kPa or less at 80 °C. 20 ml of 95% ethanol was added to the resulting residue. L*a*b* value was measured by using this menthol as a color difference measurement sample using a colorimetric color difference meter ZE-2000 (made by Nippon Denshoku Industries Co., Ltd) pursuant to ISO 7724 (JIS Z8722) to calculate chroma. Results are shown in Table 1. The purified natural (L)-menthol with the chroma being 1 or less was described as Example 6, 7, 8, 9, 10, and 11.

[Table 1]

| | Sample Name | Amount of Activated Carbon Added (%) | Stirring Time (h) | Color Measurement (Transmission) | | | | Evaluation of Smoking Taste | |
|---|---|---|---|---|---|---|---|---|---|
| | | | | L* | a* | b* | Chroma (C*) | Refreshing Feeling | Mint-like flavor |
| Preparation Example 1 | Comparative Example 1 | 0 | 0 | 97.65 | -0.25 | 2.01 | 2.02 | C | D |
| | Comparative Example 2 | 1 | 5 | 99.03 | -0.10 | 1.13 | 1.14 | C | C |
| | Example 1 | 2 | 2.5 | 99.50 | -0.08 | 0.80 | 0.80 | B | B |
| | Example 2 | 2 | 5 | 99.39 | -0.05 | 0.84 | 0.85 | B | B |
| | Example 3 | 2 | 15 | 99.63 | -0.16 | 0.62 | 0.64 | A | B |
| | Example 4 | 4 | 5 | 99.81 | -0.14 | 0.51 | 0.53 | A | A |
| Preparation Example 2 (2 x Impurities) | Comparative Example 3 | 0 | 0 | 89.72 | 0.02 | 5.05 | 5.05 | D | D |
| | Comparative Example 4 | 1 | 5 | 98.64 | -0.19 | 1.62 | 1.63 | C | D |
| | Comparative Example 5 | 2 | 2.5 | 98.34 | -0.14 | 1.44 | 1.44 | C | D |
| | Comparative Example 6 | 2 | 5 | 98.25 | -0.09 | 1.47 | 1.47 | C | D |
| | Comparative Example 7 | 2 | 15 | 98.48 | -0.06 | 1.44 | 1.44 | C | D |
| | Example 5 | 4 | 5 | 99.40 | -0.16 | 0.93 | 0.94 | B | C |
| Preparation Example 3 | Example 6 | 0 | 0 | 99.85 | -0.17 | 0.78 | 0.80 | B | B |

(continued)

| | Sample Name | Amount of Activated Carbon Added (%) | Stirring Time (h) | Color Measurement (Transmission) | | | | Evaluation of Smoking Taste | |
|---|---|---|---|---|---|---|---|---|---|
| | | | | L* | a* | b* | Chroma (C* ) | Refreshing Feeling | Mint-like flavor |
| (1/2 x Impurities) | Example 7 | 1 | 5 | 99.52 | -0.03 | 0.52 | 0.52 | A | A |
| | Example 8 | 2 | 2.5 | 99.45 | 0.00 | 0.44 | 0.44 | A | A |
| | Example 9 | 2 | 5 | 99.55 | -0.11 | 0.43 | 0.45 | A | A |
| | Example 10 | 2 | 15 | 99.63 | -0.13 | 0.42 | 0.44 | A | A |
| | Example 11 | 4 | 5 | 99.76 | -0.08 | 0.30 | 0.31 | A | B |

[Evaluation]

Trained 14 panelists performed sensory tests for refreshing feeling and mint-like flavor of a cigarette in smoking. They conducted the evaluations on a 10-point scale and further evaluated their averages as follows:

- 8.0 or more point: Grade A (very strong refreshing feeling, or very good and strong mint-like flavor),
- 7.0 or more - less than 8.0 point: Grade B (strong refreshing feeling, or good and strong mint-like flavor),
- 6.0 or more - less than 7.0 point: Grade C (slightly weak refreshing feeling or mint-like flavor),
- Less than 6.0 point: Grade D (weak refreshing feeling or mint-like flavor).

"Stirring time" in Table 1 represents the time during which the heated and melted purified natural (L)-menthol is stirred under hot bath at 60 °C after addition of the activated carbon.

From Table 1, it was found out that, generally, the more the activated carbon was added to the sample, the higher grades the refreshing feeling and mint-like flavor acquired in the evaluation of the smoking taste. However, when too much amount of activated carbon was added to the sample with respect to the amount of impurities, the evaluation of mint-like flavor fell (see Example 11).

Also, the longer the stirring time became, the stronger the refreshing feeling became. However, in Preparation Examples 2 and 3, no difference was confirmed among the evaluations by the smoking tastes depending on the stirring time.

In all Examples, there was a tendency that the lower the chroma was, the higher the evaluation of the smoking taste was. However, if the chroma becomes 0.31 or less (see Example 11), the mint-like flavor will be lost. Also, if the chroma was 1 or less, the evaluations of refreshing feeling and mint-like flavor were relatively high. And if the chroma was within the range of 0.4 to 0.6, the grades of both refreshing feeling and mint-like flavor were very high, Grade A.

In Preparation Examples 1 and 2, the more the activated carbon was added to the sample, the higher the L* value was, but there were little difference among the L* values between Examples 1 and 2. No correlation between an amount of activated carbon added and L* values was seen in Preparation Example 3.

In all Preparation Examples, the lower the b* value was, the higher the evaluation of the smoking taste was, which had similar tendencies as in the evaluation with chroma, i.e., . That is, it was found out that, when there were many impurities in the purified natural (L)-menthol, yellow color was shown in the evaluation by the color difference meter.

From above, since the chroma is correlated with the evaluation of the smoking taste, if we use the chroma as an indicator using this evaluation method, the smoking taste can be evaluated not only with sensory evaluation but also with numerical values. Also, generally, the more the activated carbon was added, the higher evaluation of the smoking taste the purified natural (L)-menthol can obtain. That is, it was found out that, if the purified natural (L)-menthol has fewer impurities, it can be highly tasty, but on the contrary, if the menthol has too few impurities, the evaluation of the smoking taste falls, especially mint-like flavor is lost. Accordingly, very small amounts of impurities are believed to be required for the natural (L)-menthol having refreshing feeling and mint-like flavor. Therefore, the method for manufacturing the purified natural (L)-menthol of the present invention can easily and inexpensively provide a purified natural (L)-menthol having refreshing feeling and mint-like flavor in light of purification conditions such as an added amount of activated carbon.

[Analysis and Measurement of Metallic Element Included in Residue]

Next, in order to confirm that the impurities are reduced or removed by activated carbon treatment, the metallic elements included in the crude menthol before the activated carbon treatment and the residues of the purified menthol after the activated carbon treatment were analyzed and measured by the following method.

5000g of the crude menthol before the activated carbon treatment and 5000g of the purified menthol after the activated carbon treatment prepared on the same condition as Example 4 of Preparation Example 1 were provided, respectively, and heated and melted at 60 °C. They were distilled with 2 kPa or less at 80 °C to give 0.396 g and 0.829g of residues, respectively. With respect to these samples, elementary analysis was conducted by ICP MS using the following reagents and measuring instruments.

[Table 2]

| Reagent Name | Grade |
|---|---|
| Nitric acid | Suprapur(Made by Merck & Co., Inc.) |
| Hydrogen peroxide | Ultrapur(Made by Merck & Co., Inc.) |
| Multi-element standard solution XVI | Certipur(Made by Merck & Co., Inc.) |

[Table 3]

| Measuring Instrument | Note |
|---|---|
| ICP MS | Thermo X series 2 (Made by Thermo Scientific, Inc.) |
| Microwave sample decomposing device | Made by Milestone Co., Ltd. |
| Detector | Mass detector |
| Data collection | Electronic data collection system |
| Carrier gas | Argon gas(super high purity) |
| Balance | Made by Sartorius AG |
| Micropipets | 1-10 microliter capacity and 10-1000 microliter capacity |

[Preparation Method of Analytical Sample]

0.1 g-0.2 g of the obtained residues were transferred to a container made by Teflon (Registered Trademark) and 5 ml of nitric acid solution was added to the residues.

Further, 1 ml of $H_2O_2$ was added to the container and the container was sealed. This container was installed in the microwave sample decomposing device to conducted a decomposition treatment for 20 minutes at 150 °C.

The container was then cooled and taken out from the microwave sample decomposing device. The residues in the container were transferred to a 100 ml measuring flask. The flask was filled up to 100 ml with ultrapure water and well shaken.

This sample solution was used for analysis and measurement of the metallic elements.

A blank without the residues added was also prepared by the above-mentioned method.

Also, standard solution for creating a calibration curve was prepared using multi-element standard solution XVI and nitric acid solution.

Specifically, 0.2% nitric acid solution was first added to 1 ml of the multi-element standard solution so that the flask was filled up to 100 ml with the solution to prepare 1000 ppb of standard stock solution.

1 ppb, 3 ppb, 5 ppb, 10 ppb, 15 ppb, and 20 ppb of standard solution were prepared using this standard stock solution and 1.0% nitric acid solution.

Measured results were converted into concentration (ppm) using the following formula.

```
{(sample read - blank read) x volume of sample solution} /

(weight of residue (g) x 1000)
```

Results are shown in Table 4.

[Table 4]

| | Element Name | Detection Limit | Before Activated Carbon Treatment | After Activated Carbon Treatment |
|---|---|---|---|---|
| 1 | Iron | 0.05 | 3670.92 | 38.74 |
| 2 | Zinc | 0.05 | 5773.62 | 154.12 |
| 3 | Sodium | 0.05 | 2640.91 | 91.08 |
| 4 | Magnesium | 0.05 | 840.48 | 13.28 |
| 5 | Aluminum | 0.05 | 1893.18 | 16.45 |
| Unit:ppm | | | | |

Table 4 shows that, if activated carbon treatments are conducted, the amounts of the metallic elements decrease to 1 / 29 - 1/118 of the amounts without activated carbon treatments.

That is, the amounts of impurities are found to substantially decrease due to the activated carbon treatments.

Thus, the large amounts of impurities containing metallic elements, such as the above-mentioned iron, zinc, sodium, magnesium, and aluminum, are removable from the menthol by the addition and purification of the activated carbon.

Accordingly, the method for manufacturing the purified natural (L)-menthol of the present invention can easily and inexpensively provide a purified natural (L)-menthol with little impurities by adding the activated carbon to the menthol.

[INDUSTRIAL APPLICABILITY]

The method for evaluating the purified natural (L)-menthol is conveniently utilized as one of the components included in the modifier for tobacco flavor and taste and in checking a degree of purification thereof.

**Claims**

1. A method for manufacturing a purified natural (L)-menthol, comprising; a step of heating and melting crude menthol containing 90% or more of the purified natural (L)-menthol to obtain a liquid crude menthol, a step of adding activated carbon to the liquid crude menthol, and a step of stirring the obtained liquid menthol to make the activated carbon absorb impurities in the crude menthol.

2. The method for manufacturing a purified natural (L)-menthol according to claim 1, further comprising; a step of filtering the obtained liquid menthol with the activated carbon absorbing the impurities to remove the activated carbon and a step of cooling down and recrystallizing the menthol.

**Patentansprüche**

1. Verfahren zur Herstellung eines gereinigten natürlichen (L)-Menthols, umfassend: einen Schritt des Erwärmens und Schmelzens von rohem Menthol, das 90% oder mehr des gereinigten natürlichen (L)-Menthols enthält, um ein flüssiges rohes Menthol zu erhalten, einen Schritt des Hinzufügens von Aktivkohle zu dem flüssigen rohen Menthol, und einen Schritt des Rührens des erhaltenen flüssigen Menthols, um die Aktivkohle Verunreinigungen in dem rohen Menthol absorbieren zu lassen.

2. Verfahren zur Herstellung eines gereinigten natürlichen (L)-Menthols nach Anspruch 1, ferner umfassend: einen Schritt zum Filtern des erhaltenen flüssigen Menthols mit der Aktivkohle, die die Verunreinigungen absorbiert, um die Aktivkohle zu entfernen, und einen Schritt zum Abkühlen und Rekristallisieren des Menthols.

**Revendications**

1. Procédé de fabrication d'un (L)-menthol naturel purifié, comprenant: une étape de chauffage et de fusion du menthol brut contenant 90 % ou plus du (L)-menthol naturel purifié pour obtenir un menthol brut liquide, une étape d'addition de charbon actif au menthol brut liquide, et une étape de brassage du menthol liquide obtenu pour que le charbon actif absorbe les impuretés dans le menthol brut.

2. Procédé de fabrication d'un (L)-menthol naturel purifié selon la revendication 1, comprenant en outre: une étape de filtration du menthol liquide obtenu avec le charbon actif absorbant les impuretés pour éliminer le charbon actif et une étape de refroidissement et recristallisation du menthol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3935987 B
- JP 5014846 B
- US 2008228013 A